(19) Europäisches Patentamt
European Patent Office
Office européen des brevets

(11) Publication number: 0 018 040 A1

(12) # EUROPEAN PATENT APPLICATION

(21) Application number: 80200321.0

(22) Date of filing: 09.04.80

(51) Int. Cl.³: C 07 D 303/24, C 07 D 301/28

(30) Priority: 19.04.79 GB 7913630

(43) Date of publication of application: 29.10.80 Bulletin 80/22

(84) Designated Contracting States: BE CH DE FR GB IT LI NL

(71) Applicant: SHELL INTERNATIONALE RESEARCH MAATSCHAPPIJ B.V., Carel van Bylandtlaan 30, NL-2596 HR DEN HAAG (NL)

(72) Inventor: Van Gogh, Johan, Badhuisweg 3, NL-1031 CM Amsterdam (NL)

(74) Representative: Keuzenkamp, Abraham et al, c/o Shell Internationale Research Maatschappij B.V. P.O. Box 302, NL-2501 CH 's-Gravenhage (NL)

(54) **Process for the preparation of polyglycidyl ethers of polyhydric phenols and polyglycidyl ethers so prepared.**

(57) Liquid polyglycidyl ethers of polyhydric phenols are prepared by (A) reacting the polyhydric phenol and an excess of epichlorohydrin in the presence of a condensation catalyst with formation of chlorohydrin ethers by addition reactions and glycidyl ethers and dichlorohydrin by transepoxidation reactions, until at least 95% of the phenolic groups have reacted, (B) distilling off epichlorohydrin and dichlorohydrin, with addition of supplementary dichlorohydrin, at temperatures at which glycidyl ethers are reconverted into chlorohydrin ethers by reaction with dichlorohydrin, (C) dissolving the residue after removal of volatile components in organic solvent and dehydrohalogenating with aqueous alkali metal hydroxide with separation of the aqueous alkali chloride solution, and isolating the polyglycidyl ether.

EP 0 018 040 A1

0018040

# PROCESS FOR THE PREPARATION OF POLYGLYCIDYL ETHERS OF POLYHYDRIC PHENOLS AND POLYGLYCIDYL ETHERS SO PREPARED

The invention relates to a process for the preparation of polyglycidyl ethers of polyhydric phenols wherein the polyhydric phenol is reacted with from 2.5 to 10 moles of epichlorohydrin per phenolic hydroxyl equivalent in the presence of a condensation catalyst and the product is dehydrohalogenated with an alkali metal hydroxide. The large excess of epichlorohydrin is required to obtain glycidyl ethers of low molecular weight, and to avoid as much as possible formation of polymeric glycidyl ethers (see formula III).

While taking 2,2-bis(4-hydroxyphenyl)propane as the polyhydric phenol and sodium hydroxide as the alkali metal hydroxide the theoretical reaction scheme can be represented by the equations:

(I)

(II)

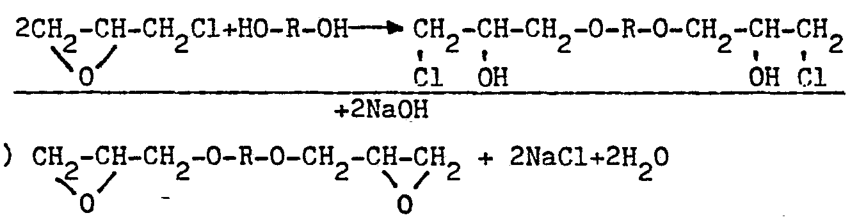


wherein -R- is the hydrocarbon residue of the phenolic compound, that is here the group of formula:

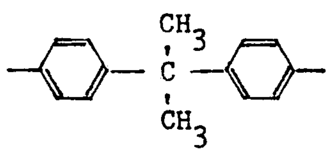

Reaction (I) is then termed the condensation reaction, and reaction (II) the dehydrohalogenation reaction. Catalysts such as tertiary amines, quaternary ammonium salts or phosphonium salts are often added, to accelerate reaction (I).

However, due to the high reactivity of the components, side reactions occur, which in practice can cause

(1) loss of epichlorohydrin by hydrolysis or polymerization,

(2) formation of polyepoxides of higher molecular weight

(III) 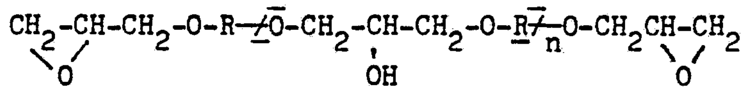

wherein n is a number greater than 0,

(3) hydrolysis of epoxy groups of glycidyl ethers with formation of terminal glycol groups in the product, and so on.

In many processes proposed the main dehydrohalogenation is carried out while the excess epichlorohydrin is still present, and sometimes use is even made of this epichlorohydrin to remove water (formed by reaction (II), and/or added with the alkali metal hydroxide) by azeotropic distillation. For example, British patent 1,278,737 discloses a process for the preparation of polyglycidyl ethers with a low molecular weight by reacting in a first stage a polyhydric phenol catalytically with an excess of epichlorohydrin, and dehydrochlorinating the product in a second stage in the presence of an excess of epichlorohydrin with an aqueous solution of 0.80 to 0.99 equivalents of an alkaline compound per original phenolic equivalent while distilling off water azeotropically, after which the epichlorohydrin is distilled off and the residue is subjected to a second dehydrochlorination with an excess of alkaline compound, this is done to keep the epichlorohydrin loss as low as possible, and to obtain high-quality polyglycidyl ethers with a low polymer content in a short time. Drawback here is that alkali chloride formed during the main dehydrohalogenation reaction is present as a solid in the reaction product and in the crude resin, and has to be removed by filtration and/or washing with water.

A further complication is the "transepoxidation" reaction, whereby a chlorohydrin ether and epichlorohydrin react in the presence of a condensation catalyst with formation of dichlorohydrin (1,3-dichloro-2-propanol) and the corrresponding glycidyl ether, according to the schematic

equation

(IV) 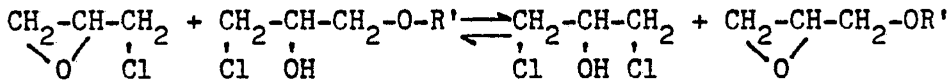

wherein R' is the rest of the molecule of the ethers.

It has been proposed to distill off the excess of epichlorohydrin (and other volatile components) after a condensation step in which substantially all the polyhydric phenol has been etherified catalytically with formation of chlorohydrin ether (see equation I above), and to dehydrohalogenate the residue with an alkali metal hydroxide (USA 2,943,095 and USA 2,943,096); the temperature in the condensation step, however, should then not exceed 60°C to avoid formation of undesirable polymeric compounds, and the long reaction times (17 hours and more) required for complete conversion of the phenolic compound at these rather low temperatures make the process unsuitable for industrial practice. Further, transepoxidation occurs with formation of dichlorohydrin and glycidyl ether (see equation (IV) above), and this dichlorohydrin has to be converted back into epichlorohydrin; this was performed according to these disclosures by either treating the crude epichlorohydrin distillate with sodium hydroxide or lime, or by treating the total product of the condensation step first with from 50-70 percent of the stoichiometric amount of base required for complete dehydrohalogenation before the epichlorohydrin was distilled off. It is clear that the transepoxidation reaction whereby dichlorohydrin is formed introduced the necessity for a stage which should be avoided, to know a stage wherein an epichlorohydrin-containing material is contacted with a base; this contact provokes hydrolysis of epichlorohydrin with formation of glycerol, glycidol, and glycidol polymers.

British patent 1,159,530 discloses a process for the preparation of glycidyl ethers of polyhydric phenols wherein the polyhydric phenol is reacted with an excess

of epichlorohydrin in the presence of a condensation catalyst at a temperatures of from 60 to 165°C for a sufficient time to obtain an equilibrium mixture of chlorohydrin ether, glycidyl ether, epichlorohydrin an dichlorohydrin, and this mixture is then dehydrohalogenated with at least the stoichiometric amount of alkali, whereupon the excess of epichlorohydrin is removed by distillation. The products, however, have chlorine contents of more than 1%w, which is undesirable for many applications. Here again epichlorohydrin is in contact with the alkaline material for some time at elevated temperature, which may easily result in losses by hydrolysis and polymerization.

We have now found a novel process, in which contact of epichlorohydrin with alkaline compounds can be completely avoided. In the novel process use is made of the fact that transepoxidation according to equation (IV) occurs, and that it is an equilibrium reaction with reaction rates large enough for potential use of this reaction. When epichlorohydrin (boiling at 117°C at atmospheric pressure) is distilled off selectively, the dichlorohydrin (boiling at 174°C at atmospheric pressure) will remain in the liquid, and under influence of the condensation catalyst will react with glycidyl ether to regenerate chlorohydrin ether and epichlorohydrin - the latter can again be removed by distillation. To accelerate the reaction and to complete the regeneration of chlorohydrin ethers, additional dichlorohydrin is now added during this distillation step.

The invention is therefore defined as a process for the preparation of a polyglycidyl ether of a polyhydric phenol wherein

(a) the polyhydric phenol is reacted with from 2.5 to 10 moles of epichlorohydrin per phenolic hydroxyl equivalent in the presence of a condensation catalyst,

(b) the excess of volatile components is removed by distillation, and

(c) the residue is dehydrohalogenated with an aqueous solution of an alkali metal hydroxide and the polyglycidylether is recovered,

which process is characterized in that

(A) the reaction of the polyhydric phenol with the epichlorohydrin is carried out in the presence of a non-volatile condensation catalyst at temperatures above 60$^{o}$C until at least 95% of the phenolic hydroxyl groups have reacted,

(B) epichlorohydrin and dichlorohydrin are distilled off with addition of dichlorohydrin at temperatures at which glycidyl ethers formed in step (A) by transepoxidation react with dichlorohydrin to form chlorohydrin ethers and epichlorohydrin, and

(C) the residue is dissolved in a volatile organic solvent and dehydrohalogenated in one or more stages with an aqueous solution of an alkali metal hydroxide, while separating the aqueous phase containing the alkali metal chloride formed after each stage.

The process can be performed batchwise or continuously.

The polyhydric phenol for use in step (A) is preferably a dihydric phenol, and more preferably a diphenylol alkane of the general formula

(V)

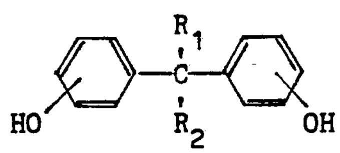

wherein $R_1$ and $R_2$ each represent a hydrogen atom or an $C_1$ to $C_6$ alkyl group. Preferably the hydroxyl groups in formula(V) are both in para-position with respect to the alkylene group. Examples are diphenylol propane [Bisphenol A; 2,2-bis(4-hydroxyphenyl)propane], diphenylol ethane, diphenylol methane (Bisphenol F), and mixtures thereof, such as mixtures of the Bisphenols A and F, for example in a 70:30 weight ratio. Polyhydric phenols with more than 2, for example 3, 4 or 5 phenolic

hydroxyl groups per molecule may also be used; examples are tetraphenylol ethane/technical 1,1,2,2-tetra(4-hydroxyphenyl)ethane/ and novolacs. The preferred polyhydric phenol is diphenylol propane.

The amount of epichlorohydrin in step (A) is from 2.5 to 10 moles, preferably from 3.5 to 8 moles, per phenolic equivalent of the polyhydric phenol.

The condensation catalyst should be non-volatile under reaction conditions of step (A), or if a volatile starting material is used, it should be converted into a non-volatile catalytically active material: a tertiary amine for example, can form a hydrochloride as catalytically active material. Preferred condensation catalysts are quaternary ammonium halides, quaternary phosphonium halides, and alkali metal halides; condition is, of course, that the solubility in the organic liquid is sufficient for a catalytic activity. Suitable quaternary ammonium salts are, for example, benzyldimethylammonium chloride, tetramethylammonium chloride and tetraethylammonium bromide; examples of quaternary phosphonium halides are ethyltriphenylphosphonium bromides and -iodide. Lithium chloride, potassium chloride, and potassium bromide are examples of suitable alkali metal halides. Such catalytically active halides can also be formed by starting with the corresponding hydroxide, which under reaction conditions forms the halide.

The condensation catalyst is preferably used in amounts of from 0.5 to 5 molar percents calculated on the polyhydric phenol.

The temperature in step (A) should be above 60°C, to ensure a sufficient conversion in a reasonable reaction time, say up to 3 hours. A reaction temperature of from 100 to 135°C is preferred. Temperatures above 140°C should be avoided to prevent side reactions and decomposition of catalyst.

A conversion of at least 95% of the phenolic hydroxyl

groups into ether groups is essential in this process to obtain final polyglycidyl ethers of desirable quality; a phenolic conversion of about 99% can usually be attained within a reasonable time. It is not necessary to aim at a fully 100% phenolic conversion, as the prolonged reaction time that involves would be out of proportion and therefore technically unattractive, and further could introduce undesirable properties in the final polyglycidyl ether, such as a high chlorine content. So in an experiment in which the phenolic conversion was 99.0% after 2.5 hours, it took 6 hours to attain a phenolic conversion of 99.9%, and the chlorine content of that final polyglycidyl ether was more than 1% by weight.

The condensation reaction may be carried out in the presence of minor amounts of water or a volatile alcohol; these compounds may have an accelerating action.

Transepoxidation reactions according to equation (IV) will occur during step (A) and will cause the presence of material amounts of dichlorohydrin and glycidyl ether compounds at the end of step (A). For example, in an experiment in which diphenylol propane and epichlorohydrin in a molar ratio of 1:10 were condensed at 120°C during 2½ hours (phenolic conversion 99%) the mixture contained 1.3 moles of dichlorohydrin per original mole of diphenylol propane.

The amount of dichlorohydrin at this stage is an important parameter in the present process. This amount can conveniently be determined by GLC analysis of the reaction mixture in the presence of a suitable marker, for example 3-chloropropanol-1.

Step (B) is a distillation step in which the excess of epichlorohydrin is distilled off, and in which also glycidyl ether components formed in step (A) by transepoxidation are largely reconverted into chlorohydrin ether components. Without added dichlorohydrin this reconversion would be either incomplete, or take too much time in the final stage of step (B), when the dichlorohydrin concentration in the liquid phase would be rather low; the long residence time of the residue at the destillation temperature would then promote side reactions which would raise epoxy molar weight, viscosity, and chlorine content of the final resin.

Therefore dichlorohydrin is added to the reaction mixture in step (B), in one or more stages, batchwise or continuously. The total amount of dichlorohydrin added in step (B) is preferably from 0.7 to 2 times the amount of dichlorohydrin present at the end of step (A); smaller or larger amounts of epichlorohydrin may be added, but will generally not improve the efficiency of the process. "Added" dichlorohydrin means that dichlorohydrin is added over the amount present at the end of step (A). Of course, recovered dichlorohydrin can be used in further batches in a batch process or as a feed-back to step (B) in a continuous process.

The chlorohydrin may be added at various stages in step (B). In a batch process, step (B) can be performed in two stages, in the first one of which the bulk of the epichlorohydrin is distilled off, with partial reconversion of dichlorohydrin present at the end of step (A) into epichlorohydrin (see equation (IV)), whereas in the second stage the excess dichlorohydrin is added and the distillation is continued. Thereby the remaining glycidyl ether is largely reconverted into chlorohydrin ether, while the corresponding amount of epichlorohydrin is distilled off; the remaining excess of dichlorohydrin can be distilled off with this epichlorohydrin, or removed in a following distillation stage.

In a batch process part of the excess dichlorohydrin may also be added in the first stage, and the remaining amount in a second stage. In a one-stage performance of

step (B) in a batch process the total amount of dichlorohydrin can be added at once at the beginning of step(B), or continuously during the distillation, preferably in speed increasing with time.

In a continuous process step (B) is preferably carried out in one or more distillation columns for fractionated distillation wherein the final mixture of step (A) and optionally the top product of the second column are fed into the first column, and epichlorohydrin is distilled off, whereas the excess dichlorohydrin is fed into the bottom product of the first column, and that mixture fed into the top of a second column for fractionated distillation, in which the reconversion is largely completed, and the volatile top product is epichlorohydrin or a mixture of epichlorohydrin and dichlorohydrin; dichlorohydrin in the bottom product of this second column can then be removed in a flash distillation unit, followed by a film evaporator to remove as much dichlorohydrin as possible. The two fractionation columns as described may also be combined into one fractionation column, wherein the excess dichlorohydrin is added at a convenient distance from the bottom.

During step (B) reconversion by the transepoxidation takes place under the influence of the condensation catalyst present in the effluent of step (A); the temperature of the liquid phase in step (B) during reconversion stages should not be above 140°C, to avoid side reactions, and decomposition of the catalyst: 135°C is a practical upper limit. More catalyst may be added, of course, for example if by overheating some of the original catalyst has decomposed, or when a more accelerated reconversion is required. In a final stage of step (B) wherein the reconversion is largely completed, and only dichlorohydrin has to be removed, the temperature of the liquid phase may be higher, for example up to 160°C.

With a view to the maximum allowable temperature

the distillations in step (B) are preferably carried out under pressures which are lower than atmospheric. The distillations during reconversion stages are preferably carried out with fractionation; this will assist in the reconversion, and will permit recovery of rather pure epichlorohydrin, which can be used as a feed for step (A) in further production. In a modification of step (B) a small amount of polyglycidyl ether product (up to 8 %w) may be added with the added dichlorohydrin to convert into epichlorohydrin that amount of dichlorohydrin which would otherwise accumulate due to incomplete conversion in step (B): the reaction taking place (equation (IV)) is in fact an equilibrium reaction, and therefore the reconversion cannot be a fully 100% complete. Another means to prevent accumulation of dichlorohydrin is to react from time to time the accumulated part of the dichlorohydrin with glycidylether product with addition of catalyst, and to add that mixture to the second stage of step (B): that alternative would also be useful in a batch process. A third method to prevent excessive accumulation of dichlorohydrin comprises a partial dehydrohalogenation of a part of it by treatment with an underdose of an aqueous alkali metal hydroxide solution. In a continuous process the continuous feeding in of a small part of the glycidylether product into step (B) would be preferred.

The liquid residue of step (B) is a chlorohydrin ether (resin precursor) which contains rather small amounts of glycidyl ethers and, when the volatile have been stripped off efficiently, only traces of dichlorohydrin.

This residue of step (B) is subjected to dehydrohalogenation (step (C)). Preferably the residue while still hot from the final distillation step is dissolved in the solvent without delay, to minimize residence time at high temperature. The solvent may be an aliphatic ketone having up to 6 carbon atoms per molecule, such as acetone, methyl

ethyl ketone, or methyl isobutyl ketone, an aliphatic alcohol having up to 6, preferably up to 4 carbon atoms per molecule, such as methanol, ethanol, propanol, iso-propanol, butanol, iso-butanol, sec.-butylalcohol, a liquid aromatic hydrocarbon having up to 8 carbon atoms per molecule, such as toluene and xylenes or a mixture of such solvents. The solvent may contain small amounts of water.

A preferred solvent is a mixture of a ketone and an alcohol as defined above, and a particularly preferred solvent is a mixture of methyl isobutyl ketone and ethanol or iso-propanol, in particular in weight ratios of from 85:15 to 50:50. Another preferred solvent is a mixture of an aliphatic alcohol and an aromatic liquid hydrocarbon as defined above, in particular a mixture of toluene and iso-propanol in weight ratios of from 85:15 to 50:50.

The amount of solvent in step (C) is preferably from 0.2 to 2, more preferably from 1 to 2, times the weight of the residue of step (B).

The temperature in step (C) is preferably kept between 60$^{o}$C and 110$^{o}$C and in the earlier stages more preferably between 70$^{o}$C and 90$^{o}$C.

The alkali metal hydroxide is preferably sodium hydroxide, and is preferably added as a 10 to 50 %w, more preferably as a 10 to 20 %w, aqueous solution, for example as a commercially available solution, if desired diluted with water. For the production of a polyglycidyl ether having a low content of saponifiable chlorine the total amount of alkali metal hydroxide should be in excess over the saponifiable chlorine in the residue of step (B), for example in an excess up to 20%.

The aqueous alkali metal hydroxide may be metered in at a constant or variable speed, or added in portions in a number of stages, in which addition of each portion in say 5 minutes is followed by a reaction time in the order of from 10 to 60 minutes with removal of the brine

after each stage. Amount and concentration of the aqueous alkali metal hydroxide in the various stages are so chosen that the alkali metal chloride formed by dehydrohalogenation dissolves in the water, and the mixture separates into two phases, an organic phase and an aqueous phase, the brine. As the reaction mixture will become heterogeneous the total mixture during addition of alkali metal hydroxide and further reaction time should be kept in turbulent motion, for example by stirring. A concentrated brine will have the greater density, and can made to settle after each reaction stage as the bottom layer, and can then be separated from the organic layer by settling means, for example by gravity settling or by centrifuging. The interlayer is usually clean by absence of polymer or gel particles, in particular when the mixture in first cooled to temperatures of from 30 to 50°C. The separated brine or brines may be steam-stripped for the recovery of organic solvent.

In a preferred performance of step (C) the residue of step (B) is dissolved in from 1 to 2 times its weight of a mixture of iso-propyl alcohol and methyl isobutyl ketone or toluene, and dehydrohalogenated in a first stage with an aqueous solution of sodium hydroxide in the presence of sufficient water to keep the sodium chloride in aqueous solution, whereupon the aqueous phase is separated and the organic phase is further dehydrohalogenated with aqueous sodium hydroxide in excess over the saponifiable chlorine still present. The advantage of carrying out step (C) by this preferred method are a fast and practically complete dehydrohalogenation, resulting in a polyglycidyl ether product having a very low content of saponifiable chlorine, for example 0.1 %w, or less.

In this preferred method the temperature during the first stage is preferably from 70 to 90°C, and during the second stage preferably from 70 to 110°C, more preferably

from 90 to 110°C.

The dehydrohalogenation with the aqueous alkali metal hydroxide may be carried out in countercurrent, in two or more stages, in which the total amount of fresh aqueous alkali is added to the last stage, and the separated aqueous layer from each stage is added to an earlier stage. In order to keep the alkalinity in the final brine low, this method may be modified by adding to the last dehydrohalogenation stage aqueous alkali in a 2 to 15 fold excess over the saponifiable chlorine, and using the aqueous layer as an alkali feed for an earlier dehydrohalogenation stage, together with such an amount of fresh alkali that the total amount of alkali metal hydroxide in the first stage is less than equivalent to the saponifiable chlorine in the residue of step (B).

The polyglycidyl ether can be recovered from the organic phase resulting from stage (C) by methods known in the art. Conveniently, the organic phase is washed with water and with a dilute aqueous solution of sodium dihydrogen phosphate, or with slightly acidified water, in order to just neutralize any residual alkali, the aqueous phase separated, and the purified organic phase flash-distilled, preferably at reduced pressure, to recover solvent as the volatile component and polyglycidyl ether as the bottom product; the solvent content of the polyglycidyl ether may then be further reduced for example in a film evaporator at reduced pressure. If desired the polyglycidyl ether or a solution thereof can be filtered by suitable means.

The liquid polyglycidyl ethers prepared by the process according to the invention have good colour, usually below 1 on the Gardner scale, low content of alpha-glycol and phenolic hydroxyl, low content of saponifiable chlorine; and polyglycidyl ethers having low viscosity can easily be prepared. Further the chemical loss of

epichlorohydrin is very low, epichlorohydrin having low dichlorohydrin content can be recovered for further use, and the liquid phases (brines, organic layer) are easy to separate. Polymer formation is so low that it can be ignored.

The invention is illustrated by examples. Parts therein are parts by weight, unless otherwise noted. Diphenylolpropane is 2,2-bis(4-hydroxyphenyl)propane. Data for epichlorohydrin/dichlorohydrin ratios were obtained by GLC analysis.

Example I

Step (A)

| | |
|---|---|
| Diphenylol propane | 228 g (1 mole) |
| Epichlorohydrin | 925 g (10 mole) |
| Tetramethylammonium chloride | 1.1 g (0.01 mole) |

are heated to 100°C in a reaction flask equipped with stirrer, thermocouple well, and reflux condenser. The temperature rose to 120-122°C (reflux temperature) by the exothermic reaction, and was kept at 120°C during 2½ hours. The phenolic conversion was 99%. The reaction mixture contained volatiles (790 g) consisting of epichlorohydrin and dichlorohydrin in a weight ratio of 78:22, and non-volatile reaction product (363 g, containing about 1,3 epoxy equivalents).

Step (B)

The volatiles (epichlorohydrin and dichlorohydrin) were distilled off in vacuum (final pressure 15 mm Hg = 2 k Pa) during 2½ hours at a maximum bottom temperature of 120°C. The distillate (746 g) contained epichlorohydrin and dichlorohydrin in a weight ratio of 92:8. The residue (405 g) had an epoxy molar mass (EMM) of 798, and contained 8.7 g of volatiles (epichlorohydrin and dichlorohydrin in a weight ratio of 1:7). The residue contained therefore 0.51 epoxy equivalents. 258 g (2 moles) of dichlorohydrin were added to the residue, and the mixture

was kept at 130°C during 1 hour. The volatiles were distilled off in vacuum (final pressure 15 mm Hg = 2 k Pa) at a bottom temperature of 130°C, during 1 hour, and remaining volatiles were removed at oil pump vacuum (1 mm Hg = 0.13 k Pa) during ½ hour at 140°C bottom temperature. The residue (408 g) had EMM 2600, and contained 0.25 %w volatiles (dichlorohydrin); the distillate (252 g) consisted of epichlorohydrin and dichlorohydrin in a weigth ratio of 1:7. The residue contained therefore 0.16 epoxy equivalents.

Step (C)

The final residue from step (B) was dissolved in 600 g of a solvent mixture consisting of methyl isobutyl ketone and iso-propanol in a 75/25 weight ratio. 510 g of a 15 %w aqueous solution of sodium hydroxide (1.91 mol Na OH) was added, and the mixture was heated with stirring at 80°C during 15 minutes. The brine was separated off, and the organic layer treated with the same amount of aqueous NaOH during 45 minutes at 80°C. The aqueous layer (mainly NaOH, and small amounts of NaCl, dissolved in water) was separated off, and stored for use as dehydrohalogenation agent for a further batch. The organic layer was washed with water (200 ml) during 15 minutes at 80°C, and then with aqueous $NaH_2PO_4$ (2 %w, 200 ml) at 80°C during 15 minutes. The solvents were distilled off, and the polyglycidyl ether heated in vacuum (15 mm Hg = 2 k Pa) during 30 minutes at 170°C to remove traces of solvent. The polyglycidylether (335 g) had the following properties:

| | |
|---|---|
| Viscosity (25°C) | 91 P |
| EMM | 183 |
| saponifiable Cl | 0.07 %w |
| total Cl | 0.6 %w |
| colour (Gardner) | 0.7 |

Step (C) was repeated, with the exception that the second dehydrohalogenation stage was carried out at 100°C during 15 minutes in an autoclave. Resin yield and properties were essentially the same, but the viscosity was slightly lower (81 P at 25°C).

This example demonstrates that in step (B) in the first distillation stage (where no dichlorohydrin has been added). the number of epoxy equivalents in the residue has been reduced from about 1.3 to 0.51, and further, that in the second distillation stage (where dichlorohydrin has been added) this number is further reduced to 0.16 epoxy equivalents. Calculations from the date given above show that these reductions of epoxy content of the residue result in increased recovery of epichlorohydrin.

The example further demonstrates that yield and quality of the polyglycidyl ethers so obtained are excellent: yield on diphenylol propane is practically quantitative, viscosity and EMM are low, the total chlorine content is low, and the saponifiable chlorine content is very low.

Example 2

Example 1 was repeated with the following exceptions: In step (B) the volatiles were distilled off in about 3 hours instead of 2½ hours, and after the dichlorohydrin was added, the mixture was kept at 130°C during 1½ hour.

The final residue of step (B) (411 g) had an EMM = 5300; the final polyglycidyl ether (335 g) had the following properties:

| | |
|---|---|
| Viscosity (25°C) | 94 P |
| EMM | 183 |
| saponifiable Cl | 0.09 %w |
| total Cl | 0.58 %w |
| colour (Gardner) | 0.7 |

Example 3

Example 2 was repeated with the following exceptions: In the second distillation the volatiles were distilled off during 1½ hour. The final residue of step (B) (412 g) had an EMM = 9000.

Further, in step (C) the weight ratio of methyl isobutyl ketone and iso-propanol was 85:15, and both dehydrohalogenation stages were carried out during 60 minutes at 80°C. The final polyglycidyl ether had the following properties:

| | |
|---|---|
| Viscosity (25°C) | 85 P |
| EMM | 186 |
| saponifiable Cl | 0.24 %w |
| total Cl | 0.88 %w |
| colour (Gardner) | 0.8 |

Example 4

Continuous preparation of a polyglycidyl ether.

Step (A).

The reactor consisted of three closed vessels in series, size 1.5 l, 3.0 l and 1.5 l respectively. The temperature was kept at 120°C. The first vessel was continuously fed be a feedstock stream comprising:

| | g/h |
|---|---|
| diphenylol propane | 374 |
| epichlorohydrin | 1,560 |
| teramethylammonium chloride | 1.9 |

The epichlorohydrin stream was made up from 301 g of fresh epichlorohydrin and 1.259 g of "recycle epichlorohydrin" obtained in step (B), and containing epichlorohydrin and dichlorohydrin in a weight ratio of 29:1.

The feedstock stream was preheated to 100°C.

The reaction mixture was continously transferred from the first to the second, and from there to the third vessel, and from there transferred to step (B).

The total residence time in step (A) was 3.5 hours. The feed to step (B) (1936 g/h) contained 13.2 %w of dichlorohydrin (2.0 mol/h) and 1.7 meq/100 g phenolic hydroxyl (phenolic conversion 99%).

Step (B)

The installation contained as main reactors two distillation columns in series, both designed for a rather large liquid hold up at a low pressure drop; each column contained a bottom sump for reaction. The reaction temperature in each column was 130°C and the pressure 0.1 atmosphere (10 k Pa). Both columns served for reaction, and for recovery of rather pure epichlorohydrin by fractionated distillation, while leaving dichlorohydrin in the residue.

The first column was continuously fed by the reaction mixture of step (A) (1936 g/h) and by the top product of the second column (491 g/h, containing 84 %w of dichlorohydrin). The distillate of the first column was the 1259 g/h of "recycle epichlorohydrin" which was used as an epichlorohydrin feed in step (A). The residue of the first column contained 0.40 glydidyl ether equivalents/hour; it was mixed in the sump with: (a) 204 g/h of recycle dichlorohydrin obtained from the dichlorohydrin strippers, this is 0.8 times the weight of dichlorohydrin present at the end of step (A), and (b) 15 g of polyglycidyl ether product obtained at the end of step (C). That mixture was fed into the second column, where reconversion of glycidyl ethers into dichlorohydrin ethers with formation of epichlorohydrin and consumption of dichlorohydrin was completed. The non-volatile component in the bottom product of the second fractionation column, mainly chlorohydrin ether component, contained 0.035 glycidyl ether equivalents/hours; the volatile component was dichlorohydrin, which contained not more than traces of epichlorohydrin. The

dichlorohydrin was recovered in a flash-distillation column followed by a film evaporator both operating in vacuum (maximum bottom temperature 160°C). The yields from this recovery stage were:

recycle dichlorohydrin: 204 g/h
resin precursor: 662 g/h, containing 0.3 %w of dichlorohydrin.

Step (C)

The resin precursor obtained in step (B) was immediately dissolved in a solvent mixture (1015 g/h) consisting of methyl isobutyl ketone, iso-propyl alcohol, and water in the weight ratio 75.5:21.2:3.3. This solvent mixture was the solvent recovered in isolating the final polyglycidyl ether product.

The resin precursor solution was dehydrohalogenated with a 15 %w aqueous sodiumhydroxide solution (NaOH:142 g, 8 % excess on phenolic equivalents in original diphenylol propane) in two stages in countercurrent, with separation of the aqueous phase in each stage. The sodium hydroxide solution was first diluted with the aqueous methyl isobutyl ketone/isopropyl alcohol/water mixture obtained by steam stripping of the final brine (from the first dehydrohalogenating stage) and the wash water before they were discarded.

The first dehydrohalogenation vessel was a 0.5 l staged reactor, operating at 85°C. It was fed continuously by the resin precursor solution (brought to 75°C)and by the aqueous phase from the second dehydrohalogenation vessel. That aqueous phase contained in solution most of the original sodium hydroxide and minor amounts of NaCl. The brine from the first dehydrohalogenator (aqueous solution of NaCl, minor amounts of NaOH, methyl isobutyl ketone, and isopropyl alcohol) was separated off, steam-stripped to recover the solvent, and discarded.

The organic phase was fed continuously into the second dehydrohalogenation vessel (a stirred reactor of 2 l, operating at 105°C) and dehydrohalogenated there to more than 99 % completion with the solvent-diluted 15 %w aqueous NaOH described above. After phase-separation the organic phase (resin solution) was washed continuously with an equal volume of acidified water, to remove traces of NaCl, NaOH, and other water-soluble compounds. Solvents were flashed off, residual volatiles removed in a film evaporator, both in vacuum at temperatures not exceeding 180°C, and the final polyglycidyl ether was cooled and stored; 15 g/h was recycled to the second stage of step (B) as indicated above. The yield of pure polyglycidyl ether was 550 g/h. It had the following properties:

| | |
|---|---|
| epoxy molar mass | 185 |
| viscosity (25°C) | 90 Poise |
| saponifiable Cl | 0.1 %w |
| total Cl | 0.6 %w |
| colour (Gardner) | 0.7 |

CLAIMS

1. Process for the preparation of a polyglycidyl ether of a polyhydric phenol wherein
(a) the polyhydric phenol is reacted with from 2.5 to 10 moles of epichlorohydrin per phenolic hydroxyl equivalent in the presence of a condensation catalyst,
(b) the excess of volatile components is removed by distillation, and
(c) the residue is dehydrohalogenated with an aqueous solution of an alkali metal hydroxide and the polyglycidylether is recovered,

which process is characterized in that
(A) the reaction of the polyhydric phenol with the epichlorohydrin is carried out in the presence of a non-volatile condensation catalyst at temperatures above 60°C until at least 95% of the phenolic hydroxyl groups have reacted,
(B) epichlorohydrin and dichlorohydrin are distilled off with addition of dichlorohydrin at temperatures at which glycidyl ethers formed in step (A) by transepoxidation react with dichlorohydrin to form chlorohydrin ethers and epichlorohydrin, and
(C) the residue is dissolved in a volatile organic solvent and dehydrohalogenated in one or more stages with an aqueous solution of an alkali metal hydroxide, while separating the aqueous phase containing the alkali metal chloride formed after each stage.

2. Process as claimed in claim 1, wherein in step (A) the polyhydric phenol and epichlorohydrin are reacted at a temperature of from 100 to 135°C.

3. Process as claimed in claim 1 or 2, wherein the condensation catalyst is a quaternary ammonium halide.

4. Process as claimed in any of claims 1 to 3, wherein the amount of dichlorohydrin added in step (B) is from

0.7 to 2.0 times the amount of dichlorohydrin present at the end of step (A).

5. Process as claimed in any of claims 1 to 4, wherein in step (B) the epichlorohydrin and dichlorohydrin are recovered by fractionated distillation.

6. Process as claimed in any of claims 1 to 5, wherein in step (C) the residue is dissolved in from 1 to 2 times its weight of a mixture of isopropyl alcohol and methylisobutyl ketone or toluene, and dehydrohalogenated in a first stage with an aqueous solution of sodium hydroxide in the presence of sufficient water to keep the sodium chloride formed in aqueous solution, whereupon the aqueous phase is separated and the organic phase is further dehydrohalogenated with aqueous sodium hydroxide in excess over the saponifiable chlorine still present.

7. Polyglycidyl ether of a polyhydric phenol, whenever prepared by a process as claimed in any of claims 1 to 6.

0018040

European Patent Office

# EUROPEAN SEARCH REPORT

Application number
EP 80 20 0321

| Category | Citation of document with indication, where appropriate, of relevant passages | Relevant to claim | CLASSIFICATION OF THE APPLICATION (Int. Cl. 3) |
|---|---|---|---|
| X | GB - A - 897 744 (SHELL)<br>* Whole patent; especially page 3; lines 14-30 *<br>--- | 1-7 | C 07 D 303/24<br>C 07 D 301/28 |
| | GB - A - 916 028 (DEVOE & RAYNOLDS)<br>* Whole patent *<br>----- | 1-7 | |

TECHNICAL FIELDS SEARCHED (Int.Cl. 3)

C 07 D 303/24
301/28

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

✓ The present search report has been drawn up for all claims

| Place of search | Date of completion of the search | Examiner |
|---|---|---|
| THE HAGUE | 16-07-1980 | ALLARD |